# EUROPEAN PATENT SPECIFICATION

(11) **EP 2 170 275 B1**
(45) Date of publication and mention of the grant of the patent: **21.11.2012**
(21) Application number: 08771808.6
(22) Date of filing: 24.06.2008
(51) Int. Cl.: A61K 9/00, A61K 31/00

(54) **Dual portion lozenge dosage form**
Lutschtabletten-dosierform mit doppelter Portion
Forme pharmaceutique en tablettte à deux parties

(30) Priority: 29.06.2007 US 947004 P; 23.06.2008 US 143916
(43) Date of publication of application: 07.04.2010
(73) Proprietor: McNeil-PPC, Inc., Skillman, NJ 08558 (US)
(72) Inventor: BUNICK, Frank, Randolph, New Jersey 07869 (US); LUBER, Joseph, Quakertown, Pennsylvania 18951 (US); WIET, Stephan, G., Morristown, New Jersey 07960 (US); MCNALLY, Gerard, Berwyn, Pennsylvania 19312 (US); WYNN, David, Huntingdon Valley, Pennsylvania 19006 (US)
(74) Representative: Kirsch, Susan Edith
(86) International application number: PCT/US2008/068001
(87) International publication number: WO 2009/006095

(56) References cited:
- WO-A-2006/061700
- US-A1- 2007 092 553

## Description

### Field of the Invention

The present invention relates to a dosage form including both a disintegrative tablet portion and a hard candy portion, and the use thereof.

### Background of the Invention

Pharmaceuticals intended for oral administration are typically provided in solid form as tablets, capsules, pills, lozenges, or granules. Rapidly disintegrative tablets are often employed in the administration of pharmaceuticals where it is impractical to provide a tablet for swallowing whole, for instance with pediatric patients. Several workers in the field have explored rapidly disintegrative tablets (e.g., U.S. Patent Nos. 6,106,861 and 6,024,981 and PCT Application No. WO 99/47126).

Further relevant prior art includes WO 2006061700 and

US 2007092553.

Applicants invention relates to a dual portion dosage form that combines the use of a rapidly disintegrative tablet containing a pharmaceutically active agent with a slower disintegrative hard candy portion (e.g., a lozenge). The dosage form, thus, provides both the benefit of the fast delivery of pharmaceutically active agent contained within the rapidly disintegrative tablet portion with the benefit of slower degrading hard candy portion, which may contain a second pharmaceutically active agent.

### Summary of the Invention

The present invention relates to a dosage form including both a disintegrative tablet portion and a hard candy portion, wherein: (i) the disintegrative tablet portion includes at least one pharmaceutically active agent, and (ii) the hard candy portion covers at least 20% of the surface of the disintegrative tablet portion, and wherein the disintegration time of the hard candy portion is at least five times (such as at least ten times) longer than the disintegration time of the disintegrative tablet portion.
Other features and advantages of the present invention will be apparent from the detailed description of the invention and from the claims.

### Detailed Description of the Invention

It is believed that one skilled in the art can, based upon the description herein, utilize the present invention to its fullest extent.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

### Disintegrative Tablet Portion

The dosage form of the present invention includes a disintegrative tablet portion. The disintegrative tablet portion includes one or more pharmaceutically active agents and optionally includes one or more compressible excipients, water-swellable excipients, effervescent couples, and other ingredients.

In one embodiment, the disintegrative tablet portion has a hardness of less than about 15 kp/cm² 98 N/cm², such as less than 10 kp/cm² 98 N/cm², such as less than 5 kp/cm²49 N/cm². In one embodiment sufficient amount of energy is applied to the disintegrative tablet portion for a sufficient amount of time to decrease its hardness. In one embodiment, energy is applied to the disintegrative tablet portion in the form of heat or electromagnetic radiation, such as microwaves. Depending on the composition of the disintegrative tablet portion, in one embodiment, heating may be performed at a temperature generally in the range of ambient temperature to 100° C or beyond for a time sufficient to achieve a softening effect.

In one embodiment, the disintegrative tablet portion has a friability of less than about 2% (such as less than about 1%, such as less than about 0.5%) prior to the application of energy to the disintegrative tablet portion, which is the second step of the process. A discussion of disintegrative tablet portion friability is presented in USP 23 (1995) 1216, p. 1981.

In one embodiment the disintegrative tablet portion is designed to dissolve in the mouth when placed on the tongue in less than about 60 seconds, e.g. less than about 45 seconds, e.g. less than about 30 seconds, e.g. less than about 15 seconds.

### Compressible Excipient

In one embodiment, the disintegrative tablet portion includes one or more compressible excipients. What is meant by a compressible excipient is an ingredient that can be compressed into a tablet shape without the addition of other binding agents. In one embodiment, the compressible excipient in the form of a hydrate, and may be selected from organic compounds such as dextrose monohydrate, maltodextrin, lactose monohydrate, and dextrin, as well as inorganic compounds including dibasic calcium phosphate dihydrate, dibasic sodium phosphate dihydrate, dibasic sodium phosphate heptahydrate, dibasic sodium phosphate dodecahydrate, monobasic sodium phosphate monohydrate, and monobasic sodium phosphate dihydrate. In one embodiment, the disintegrative tablet portion includes a compressible excipient selected from the group consisting of isomalt, dextrose monohydrate, maltodextrin, lactose monohydrate, dextrin, mannitol, lactitol, sorbitol, xylitol, erythritol, sucrose, and lactose.

In one embodiment, the compressible excipient(s) are in the form of particles having an average particle diameter of from about 50 to about 500 microns, such as from about 75 to about 400 microns.

In one embodiment, the disintegrative tablet portion includes from about 5 to about 90 percent, such as from about 15 to about 75 percent, by weight of one or more compressible excipients. In one embodiment, the disintegrative tablet portion includes at least 40 percent by weight of the one or more compressible excipients, based on the total weight of the disintegrative tablet portion.

### Water-Swellable Excipient

In one embodiment, the disintegrative tablet portion further includes one or more water-swellable excipients. What is meant be water swellable excipient is a material that is designed to swell or wick liquid upon contact with a liquid medium and to aid in the disintegration of the compressed tablet. The water-swellable excipient may be selected from superdisintegrants such as crospovidone, croscarmellose, sodium starch glycolate, cellulose compounds such as microcrystalline cellulose, starches, alginic acid and inorganic clays such as bentonite, attapulgite, and magnesium aluminum silicate. In one embodiment, the a water-swellable excipient is at least partially hydrated and selected from the group consisting of sodium starch glycolate, crospovidone, croscarmellose, microcrystalline cellulose, starches, hydroxypropyl cellulose, and alginic acid.

In one embodiment, the amount of water-swellable excipient(s) in the disintegrative tablet portion is from about 0.1 to about 5 percent by weight, such as from about 0.5 to about 3 percent by weight of the total weight of the disintegrative tablet portion.

In one embodiment, the compressible excipient(s) is present in a greater amount than the water-swellable excipient(s). In one embodiment, the ratio of compressible excipient(s) to water-swellable excipient(s) in the disintegrative tablet portion is from about 1:1 to about 150:1, such as from about 10:1 to about 100:1, such as from about 25:1 to about 75:1.

### Effervescent Couple

In one embodiment, the disintegrative tablet portion further includes one or more effervescent couples. In one embodiment, effervescent couple includes one member from the group consisting of sodium bicarbonate, potassium bicarbonate, calcium carbonate, magnesium carbonate, sodium carbonate and one member selected from the group consisting of citric acid, malic acid, fumaric acid, tartaric acid, phosphoric acid, alginic acid.

In one embodiment, the combined amount of the effervescent couple(s) in the disintegrative tablet portion is from about 0.1 to about 20 percent by weight, such as from about 2 to about 10 percent by weight of the total weight of the disintegrative tablet portion.

### Other Ingredients

The disintegrative tablet portion may include other conventional ingredients, including other fillers, which include water-soluble compressible carbohydrates such as dextrose, sucrose, mannitol, sorbitol, maltitol, xylitol, lactose, and mixtures thereof; other conventional dry binders like polyvinyl pyrrolidone and the like; sweeteners such as aspartame, acesulfame potassium, sucralose, and saccharin; lubricants, such as magnesium stearate, stearic acid, talc, and waxes; preservatives; flavors; disintegrants, antioxidants; acidulants, such as but not limited to citric acid, malic acid, tartaric acid, ascorbic acid, and fumaric acid; surfactants; and coloring agents

### Manufacture

The disintegrative tablet portion may be made in a variety of tableting methods. Conventional methods for tablet production include direct compression ("dry blending"), dry granulation followed by compression, and wet granulation followed by drying and compression. Other methods include the use of compacting roller technology such as a chilsonator or drop roller, or molding, casting, or extrusion technologies. All of these methods are well known in the art, and are described in detail in, for example, Lachman, et al., The Theory and Practice of Industrial Pharmacy, Chapter 11, (3rd Ed. 1986).

In one embodiment, the disintegrative tablet portions are formed by the direct compression method, which involves directly compacting a blend of the pharmaceutically active agent, the compressible excipient, the water-swellable excipient, and any other appropriate optional ingredients. After blending, a pre-determined volume of particles is filled into a die cavity of a rotary tablet press, which continuously rotates as part of a "die table" from the filling position to a compaction position. The particles are compacted between an upper punch and a lower punch to an ejection position, at which the resulting disintegrative tablet portion is pushed from the die cavity by the lower punch and guided to an ejection chute by a stationary "take-off" bar.

### Multiple Layers

In one embodiment, the disintegrative tablet portion has multiple layers including at least one different ingredient. In one embodiment, the disintegrative tablet portion contains two layers, wherein the first layer includes the pharmaceutically active agent and a second layer includes a second pharmaceutically active agent that is different from the pharmaceutically active agent included within the first layer. In one embodiment, disintegrative tablet portion contains two layers, wherein both the first layer and the second include the same pharmaceutically active agent and further wherein pharmaceutically active agent within the second layer is coated with a modified release coating, or is in the form of a matrix which dissolves in a modified release manner. In one embodiment the second layer includes particles of the active ingredient which are substantially coated with a modified release coating. As used herein, the term "substantially covers" means that the coating generally covers the entire surface (e.g., of the active ingredient, core or underlying layer) so that little to none of the active ingredient, core, or underlying layer is exposed. As used herein, "substantially coated" shall mean that less than about 20%, e.g. less than about 15%, or less than about 1.0% of the surface area is exposed, e.g. not covered, with a desired coating.

In one embodiment, both the first layer and the second layer are exposed on the surface of the dosage form.

In one embodiment one layer of the bi-layered disintegrative tablet portion includes one flavor and the second layer includes a different second flavor in order to sequentially deliver a flavor profile.

In one embodiment the first layer of the bilayer disintegrative tablet portion includes one immediate release active ingredient and the second layer includes an active ingredient which is the same or different from the first active ingredient and which is delivered in a modified release manner.

In one embodiment the circumference of a round compressed bilayer disintegrative tablet is surrounded by the lozenge portion, and the face of the first layer of the disintegrative tablet is exposed on the top of the dosage form and the face of the second layer of the disintegrative tablet is exposed on the bottom of the dosage form.

### Hard Candy Portion

The dosage form of the present invention includes a hard candy portion. In one embodiment, the hard candy portion is a sugar glass hard candy formed from by cooling boiled sugar candy. In another embodiment, the hard candy portion is a compressed sugar candy made by compression, with a hardness of at least 15 kiloponds, such as at least 20 kiloponds.

In one embodiment, the hard candy portion includes one or more sugars selected from the group consisting of isomalt, sucrose, lactose, dextrose, corn syrup, lactitol, and lycasin. In one embodiment, the hard candy portion includes at least 50% (such as at least 75 %, such as at least 90%) by weight of such sugar(s). In one embodiment, the hard candy portion is substantially free of sucrose (e.g., the candy portion contains isomalt or lactose).

In one embodiment, the hard candy portion has hardness of greater than about 15 kp/cm².

In one embodiment, the hard candy portion includes a pharmaceutically active agent. In one embodiment, the hard candy portion includes a pharmaceutically active agent that is different from the pharmaceutically active agent included within the disintegrative tablet portion.

The sugar glass hard candy portion can be made from a variety of methods including but not limited to uniplast rolling, roping and subsequent cutting and stamping, as well as depositing into molds. These molds can be made from metal, rubber, resin, or plastic.

Compressed sugar lozenges are made via tableting and compression techniques known in the art for making tablets, although they are compressed at hardness levels above those traditionally used for chewable, disintegrative or swallowable tablets, i.e. above 15 kiloponds, and are designed to dissolve slowly in the oral cavity.

### Pharmaceutically Active Agent

The dosage form of the present invention includes at least one pharmaceutically active agent. What is meant by a "pharmaceutically active agent" is an agent (e.g., a compound) that is permitted or approved by the U.S. Food and Drug Administration, European Medicines Agency, or any successor entity thereof, for the oral treatment of a condition or disease. Suitable pharmaceutically active agents include, but are not limited to, analgesics, anti-inflammatory agents, antihistamines, antibiotics (e.g., antibacterial, antiviral, and antifungal agents), antidepressants, antidiabetic agents, antispasmodics, appetite suppressants, bronchodilators, cardiovascular treating agents (e.g., statins), central nervous system treating agents, cough suppressants, decongestants, diuretics, expectorants, gastrointestinal treating agents, anesthetics, mucolytics, muscle relaxants, osteoporosis treating agents, stimulants, nicotine, and sedatives.

Examples of suitable gastrointestinal treating agents include, but are not limited to: antacids such as aluminum-containing active ingredients (e.g., aluminum carbonate, aluminum hydroxide, dihydroxyaluminum sodium carbonate, and aluminum phosphate), bicarbonate-containing active ingredients, bismuth-containing active ingredients (e.g., bismuth aluminate, bismuth carbonate, bismuth subcarbonate, bismuth subgallate, and bismuth subnitrate), calcium-containing active ingredients (e.g., calcium carbonate), glycine, magnesium-containing active ingredients (e.g., magaldrate, magnesium aluminosilicates, magnesium carbonate, magnesium glycinate, magnesium hydroxide, magnesium oxide, and magnesium trisilicate), phosphate-containing active ingredients (e.g., aluminum phosphate and calcium phosphate), potassium-containing active ingredients (e.g., potassium bicarbonate), sodium-containing active ingredients (e.g., sodium bicarbonate), and silicates; laxatives such as stool softeners (e.g., docusate) and stimulant laxatives (e.g., bisacodyl); H2 receptor antagonists, such as famotidine, ranitidine, cimetadine, and nizatidine; proton pump inhibitors such as omeprazole and lansoprazole; gastrointestinal cytoprotectives, such as sucraflate and misoprostol; gastrointestinal prokinetics such as prucalopride; antibiotics for H. pylori, such as clarithromycin, amoxicillin, tetracycline, and metronidazole; antidiarrheals, such as bismuth subsalicylate, kaolin, diphenoxylate, and loperamide; glycopyrrolate; analgesics, such as mesalamine; antiemetics such as ondansetron, cyclizine, diphenyhydroamine, dimenhydrinate, meclizine, promethazine, and hydroxyzine; probiotic bacteria including but not limited to lactobacilli; lactase; racecadotril; and antiflatulents such as polydimethylsiloxanes (e.g., dimethicone and simethicone, including those disclosed in United States Patent Nos. 4,906,478, 5,275,822, and 6,103,260); isomers thereof; and pharmaceutically acceptable salts and prodrugs (e.g., esters) thereof.

Examples of suitable analgesics, anti-inflammatories, and antipyretics include, but are not limited to, non-steroidal anti-inflammatory drugs (NSAIDs) such as propionic acid derivatives (e.g., ibuprofen, naproxen, ketoprofen, flurbiprofen, fenbufen, fenoprofen, indoprofen, fluprofen, pirprofen, carprofen, oxaprozin, pranoprofen, and suprofen) and COX inhibitors such as celecoxib; acetaminophen; acetyl salicylic acid; acetic acid derivatives such as indomethacin, diclofenac, sulindac, and tolmetin; fenamic acid derivatives such as mefanamic acid, meclofenamic acid, and flufenamic acid; biphenylcarbodylic acid derivatives such as diflunisal and flufenisal; and oxicams such as piroxicam, sudoxicam, isoxicam, and meloxicam; isomers thereof; and pharmaceutically acceptable salts and prodrugs thereof.

Examples of antihistamines and decongestants, include, but are not limited to, bromopheniramine, chlorcyclizine, dexbrompheniramine, bromhexane, phenindamine, pheniramine, pyrilamine, thonzylamine, pripolidine, ephedrine, phenylephrine, pseudoephedrine, phenylpropanolamine, chlorpheniramine, dextromethorphan, diphenhydramine, doxylamine, astemizole, terfenadine, fexofenadine, naphazoline, oxymetazoline, montelukast, propylhexadrine, triprolidine, clemastine, acrivastine, promethazine, oxomemazine, mequitazine, buclizine, bromhexine, ketotifen, terfenadine, ebastine, oxatamide, xylomeazoline, loratadine, desloratadine, and cetirizine; isomers thereof; and pharmaceutically acceptable salts and esters thereof.

Examples of cough suppressants and expectorants include, but are not limited to, diphenhydramine, dextromethorphan, noscapine, clophedianol, menthol, benzonatate, ethylmorphone, codeine, acetylcysteine, carbocisteine, ambroxol, belladona alkaloids, sobrenol, guaiacol, ambroxol, and guaifenesin; isomers thereof; and pharmaceutically acceptable salts and prodrugs thereof.

Examples of muscle relaxants include, but are not limited to, cyclobenzaprine and chlorzoxazone metaxalone, and orphenadrine, methocarbamol; isomers thereof; and pharmaceutically acceptable salts and prodrugs thereof.

Examples of stimulants include, but are not limited to, caffeine.

Examples of sedatives include, but are not limited to sleep aids such as antihistamines (e.g., diphenhydramine), eszopiclone, and zolpidem; isomers thereof; and pharmaceutically acceptable salts and prodrugs thereof.

Examples of appetite suppressants include, but are not limited to, phenylpropanolamine , phentermine, and diethylcathinone; isomers thereof; and pharmaceutically acceptable salts and prodrugs thereof

Examples of anesthetics (e.g., for the treatment of sore throat) include, but are not limited to dyclonene, benzocaine, and pectin; isomers thereof; and pharmaceutically acceptable salts and prodrugs thereof.

Examples of suitable statins include but are not limited to atorvastin, rosuvastatin, fluvastatin, lovastatin, simvustatin, atorvastatin, pravastatin; isomers thereof; and pharmaceutically acceptable salts and prodrugs thereof.

In one embodiment, the pharmaceutically active agent included within the disintegrative tablet portion is selected from phenylephrine, dextromethorphan, ambroxol, pseudoephedrine, acetaminophen, ibuprofen, ketoprofen, loperamide, famotidine, calcium carbonate, simethicone, and menthol; isomers thereof; and pharmaceutically acceptable salts and prodrugs thereof.

In one embodiment, the pharmaceutically active agent included within the hard candy portion is selected from phenylephrine, dextromethorphan, ambroxol, pseudoephedrine, chlorpheniramine, methocarbomal, chlophedianol, ascorbic acid, menthol, pectin, dyclonine, and benzocaine; isomers thereof; and pharmaceutically acceptable salts and prodrugs thereof.

As discussed above, the pharmaceutically active agents of the present invention may also be present in the form of pharmaceutically acceptable salts, such as acidic/anionic or basic/cationic salts. Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphospate, polygalacturonate, salicylate, stearate, subacetate, succinate, sulfate, tannate, tartrate, teoclate, tosylate and triethiodide. Pharmaceutically acceptable basic/cationic salts include, and are not limited to aluminum, benzathine, calcium, chloroprocaine, choline, diethanolamine, ethylenediamine, lithium, magnesium, meglumine, potassium, procaine, sodium and zinc.

As discussed above, the pharmaceutically active agents of the present invention may also be present in the form of prodrugs of the pharmaceutically active agents. In general, such prodrugs will be functional derivatives of the pharmaceutically active agent, which are readily convertible in vivo into the required pharmaceutically active agent. Conventional procedures for the selection and preparation of suitable prodrug derivatives are described, for example, in "Design of Prodrugs", ed. H. Bundgaard, Elsevier, 1985. In addition to salts, the invention provides the esters, amides, and other protected or derivatized forms of the described compounds.

Where the pharmaceutically active agents according to this invention have at least one chiral center, they may accordingly exist as enantiomers. Where the pharmaceutically active agents possess two or more chiral centers, they may additionally exist as diastereomers. It is to be understood that all such isomers and mixtures thereof are encompassed within the scope of the present invention. Furthermore, some of the crystalline forms for the pharmaceutically active agents may exist as polymorphs and as such are intended to be included in the present invention. In addition, some of the pharmaceutically active agents may form solvates with water (e.g., hydrates) or common organic solvents, and such solvates are also intended to be encompassed within the scope of this invention.

In one embodiment, the pharmaceutically active agent or agents are present in the dosage form in a therapeutically effective amount, which is an amount that produces the desired therapeutic response upon oral administration and can be readily determined by one skilled in the art. In determining such amounts, the particular pharmaceutically active agent being administered, the bioavailability characteristics of the pharmaceutically active agent, the dose regime, the age and weight of the patient, and other factors must be considered, as known in the art.

The pharmaceutically active agent may be present in various forms. For example, the pharmaceutically active agent may be dispersed at the molecular level, e.g. melted, within the dosage form, or may be in the form of particles, which in turn may be coated or uncoated. If the pharmaceutically active agent is in form of particles, the particles (whether coated or uncoated) typically have an average particle size of from about 1 to about 2000 microns (e.g., from about 1 to about 1000 microns). In one embodiment, such particles are crystals having an average particle size of from about 1 to about 300 microns. In another embodiment, the particles are granules or pellets having an average particle size of from about 50 to about 2000 microns, such as from about 50 to about 1000 microns, such as from about 100 to about 800 microns.

If the pharmaceutically active agent has an objectionable taste, the pharmaceutically active agent may be coated with a taste masking coating, as known in the art. Examples of suitable taste masking coatings are described in U.S. Patent No. 4,851,226, U.S. Patent No. 5,075,114, and U.S. Patent No. 5,489,436. Commercially available taste masked pharmaceutically active agents may also be employed. For example, acetaminophen particles which are encapsulated with ethylcellulose or other polymers by a coaccervation process may be used in the present invention. Coaccervation-encapsulated acetaminophen may be purchased commercially from Eurand America, Inc. (Vandalia, Ohio) or from Circa Inc. (Dayton, Ohio).

The pharmaceutically active agent may be present in pure crystal form or in a granulated form prior to the addition of the coating (e.g., modified release or taste masking coating). Granulation techniques may be used to improve the flow characteristics or particle size of the pharmaceutically active agent to make it more suitable for compression or subsequent coating. Suitable binders for making the granulation include but are not limited to starch, polyvinylpyrrolidone, polymethacrylates, hydroxypropylmethylcellulose, and hydroxypropylcellulose. The particles including pharmaceutically active agent(s) may be made by cogranulating the pharmaceutically active agent(s) with suitable substrate particles via any of the granulation methods known in the art. Examples of such granulation method include, but are not limited to, high sheer wet granulation and fluid bed granulation such as rotary fluid bed granulation, the details of which are disclosed in, "The Theory and Practice of Industrial Pharmacy, 3rd edition", Chapter 11, Lachman, Leon et. al, 1986.

In one embodiment, the pharmaceutically active agent is coated with a combination of a water insoluble film forming polymer (such as but not limited to cellulose acetate or ethylcellulose) and a water-soluble polymer (such as but not limited to povidone, polymethacrylic co-polymers such as those sold under the tradename Eudragit E-100 from Rohm America, and hydroxypropylcellulose). In this embodiment, the ratio of water insoluble film forming polymer to water soluble polymer is from about 50 to about 95 percent of water insoluble polymer and from about 5 to about 50 percent of water soluble polymer, and the weight percent of the coating by weight of the coated taste-masked particle is from about 5 percent to about 40 percent.

In one embodiment one or more active ingredients or a portion of the pharmaceutically active ingredient may be bound to an ion exchange resin in the disintegrative tablet portion or the lozenge portion for the purposes of taste-masking the pharmaceutically active ingredient or delivering the active in a modified release manner.

In one embodiment, the pharmaceutically active agent is capable of dissolution upon contact with a fluid such as water, stomach acid, intestinal fluid or the like. In one embodiment, the dissolution characteristics of the pharmaceutically active agent within the disintegrative tablet portion meets USP specifications for immediate release tablets including the pharmaceutically active agent. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999). In another embodiment, the dissolution characteristics of the pharmaceutically active agent are modified: e.g. controlled, sustained, extended, retarded, prolonged, delayed and the like.

### Salvation Inducing Agent

In one embodiment the disintegrative tablet portion, the lozenge portion or both include one or more salivation inducing agents. Examples of suitable salivation inducing agents include, but are not limited to, muscarinic acetylcholine receptor agonists (such as pilocarpine and a succulence agent, which is commercially available from IFF under the tradename SN12011), sigma binders such as arylalkylamines (e.g., N, N-disubstituted phenylalkylamines wherein the alkyl has from about 1 to about 8 carbons), N,N disubstituted-2-phenylcyclopropylamines, spirooxathiolane-quinnuclidine, Heliopsis longpipes root, and cholinesterase inhibitors. In one embodiment, the disintegrative tablet portion and/or lozenge portion includes a salivation inducing agent in an amount from about 0.1 % to about 10% by weight of the disintegrative tablet portion.

### Dual Portion Dosage Forms

In one embodiment, the weight ratio between the disintegrative tablet portion and a hard candy portion is from about 10:90 to about 60:40. In one embodiment, the hard candy portion covers at least 20 percent of the surface area of the disintegrative tablet portion (e.g., at least 50 percent or at least 75 percent or substantially covers all of the surface area of the disintegrative tablet portion). In one embodiment, the hard candy portion includes a plurality of openings exposing the surface area of the disintegrative tablet portion. In one embodiment, the hard candy portion substantially covers all of the surface area of the disintegrative tablet portion and wherein the hard candy portion further includes a plurality of indentations that, upon contact with the fluids in the oral cavity, are adapted to dissolve and expose the surface area of the disintegrative tablet portion.

In one embodiment, the hard candy portion includes an pharmaceutically active agent different from the pharmaceutically active agent included within the disintegrative tablet portion.

In one embodiment, the dosage form has a multiple layer structure, wherein the disintegrative tablet portion is one layer and the hard candy portion is the other layer. In one embodiment, the face of the first layer has a convex shape and the face of the second layer has a concave shape. In a further embodiment, the dosage form further includes a third layer positioned between a face of the first layer and a face of the second layer, wherein the third layer includes an edible adhesive-like material. In one embodiment, the edible adhesive-liker material includes an ingredient selected from the group consisting of polyethylene glycol, polyethylene oxide, polycaprolactone, camuba wax, microcrystalline wax, oppanol, shellac wax, and beeswax.

In one embodiment, the edible adhesive is pre-melted at between about 35°C to about 100°C and added to one face of the lozenge portion or one face of the disintegrative tablet portion, and allowed to cool and harden at room temperature (e.g., about 25°C).

In another embodiment, the adhesive is added as a powder between the disintegrative tablet portion and the lozenge portion and heated in a separate step for at least 2 seconds between about 35°C and about 120°C, and allowed to cool and harden at room temperature.

In another embodiment, the adhesive is applied by first preparing a sugar and/or a polymer (such as but not limited to polymethacrylates, hydroxypropylmethylcellulose, hydroxypropylcellulose, starch, and polyvinyl pyrrolidone) in water solution, then placing about 0.1 mL to about 5 mL of the solution (e.g., prepared at about 1 percent to about 50 percent solids) to one face of the lozenge, then adding the disintegrative tablet portion to that face of the lozenge and then allowing the dosage form to dry.

The edible adhesive can be added to the dosage form from about 0.05 percent to about 40 percent, e.g. from about 0.5 percent to about 10 percent by weight of the total weight of the dosage form.

In one embodiment, the pharmaceutically active agent included within the disintegrative tablet portion is selected from the group consisting of phenylephrine, dextromethorphan, ambroxol, pseudoephedrine, acetaminophen, ibuprofen, ketoprofen, loperamide, famotidine, calcium carbonate, simethicone, and menthol, and pharmaceutically acceptable salts or prodrugs thereof.

In one embodiment, the pharmaceutically active agent included within the hard candy portion is selected from the group consisting of phenylephrine, dextromethorphan, ambroxol, pseudoephedrine, chlorpheniramine, methocarbomal, chlophedianol, ascorbic acid, menthol, pectin, dyclonine, and benzocaine, and pharmaceutically acceptable salts or prodrugs thereof.

### Disintegration Test

The disintegration time of the hard candy portion of the dosage form is at least ten times, such as at least 50 times or at least 100 times, longer than the disintegration time of the disintegrative tablet portion. To determine the disintegration for the hard candy portion and the disintegrative tablet portion, the disintegration test for "Uncoated Tablets" according to USP30-NF25 (using water as the immersion fluid) should be use. Briefly, one dosage unit is placed in each of the six tubes of the basket, and water (maintained at 37 ± 2 C) is used as the immersion fluid. The disintegration time is determined by taking the average of ten measurements of the time period required to completely disintegrate the respective tablet portion. In one embodiment, the disintegration time of the disintegrative tablet portion is less than about 30 sec, such as less than about 15 sec.

### Hardness Test

Hardness is a term used in the art to describe the diametral breaking strength as measured by a Schleuniger Hardness Tester as described in Leiberman et al., Pharmaceutical Dosage Forms - Tablets, Volume 2, 2nd ed., Marcel Dekker Inc., 1990, pp. 213 - 217, 327 - 329. In order to perform the hardness test a single tablet is placed into the steel chamber within the hardness tester, and the steel piston pushes against the dosage form until it breaks, measuring the force applied as a hardness measurement. In general, 5 tablets are tested from any one sample in order to provide a mean hardness value in kiloponds.

### Sweetness

As used herein, "sweetness index" is a term used to describe the level of sweetness of the disintegrative tablet portion, the lozenge portion or the entire dosage form relative to sucrose. Sucrose, defined as the standard, has a sweetness index of 1. For example, the sweetness indices of several known sweetener compounds are listed below:

| | |
|---|---|
| Sorbitol | 0.54 - 0.7 |
| Dextrose | 0.6 |
| Mannitol | 0.7 |
| Sucrose | 1.0 |
| High Fructose Corn Syrup 55% | 1.0 |
| Xylitol | 1.0 |
| Fructose | 1.2 - 1.7 |
| Cyclamate | 30 |
| Aspartame | 180 |
| Acesulfame K | 200 |
| Saccharin | 300 |
| Sucralose | 600 |
| Talin | 2000 - 3000 |

In one embodiment, the disintegrative tablet portion and/or lozenge of the dosage form of the present invention has a sweetness index less than about 0.6. If a higher sweetness is desired, the addition of sweetening agent may increase the sweetness of the dosage form to at least about 0.9, e.g. at least about 1.0, at least about 1.5, or at least about 2.0.

### Use of Dosage Form

In one embodiment, the present invention features a method of treating an ailment, the method including orally administering the above described dosage form wherein the dosage form includes an amount of the pharmaceutically active agent effective to treat the ailment. Examples of such ailments include, but are not limited to, pain (such as headaches, migraines, sore throat, cramps, back aches and muscle aches), fever, inflammation, upper respiratory disorders (such as cough and congestion), infections (such as bacterial and viral infections), depression, diabetes, obesity, cardiovascular disorders (such as high cholesterol, triglycerides, and blood pressure), gastrointestinal disorders (such as nausea, diarrhea, irritable bowel syndrome and gas), sleep disorders, osteoporosis, and nicotine dependence.

In one embodiment, the method is for the treatment of an upper respiratory disorder, wherein the pharmaceutically active agent is selected from the group of phenylephrine, cetirizine, loratadine, fexofenadine, diphenhydramine, dextromethorphan, chlorpheniramine, chlophedianol, and pseudoephedrine and the hard candy portion includes an pharmaceutically active agent selected from the group of menthol, dyclonine, pectin, and benzocaine.

### Examples

Specific embodiments of the present invention are illustrated by way of the following examples.

### Example 1: Preparation of Disintegrative Tablet Portion Containing Dextromethorphan

### Part A: Preparation of Layered Dextromethorphan

First, an aqueous solution is prepared containing the following three ingredients: dextromethorphan hydrobromide (20% by weight); polyvinyl pyrrolidone (1% by weight); and deionized water (79% by weight).

Then, 1.96 kg of microcrystalline cellulose (Avicel PH 200 Grade, commercially available from FMC Corporation, Philadelphia, PA) is charged into a fluidized bed coating apparatus (Glatt Model GPCG 5/9, commercially available from Glatt Air Techniques, Binzen, Germany) fitted with a rotor (tangential spray) attachment. The microcrystalline cellulose is fluidized with an airflow at 36°C, and the above dextromethorphan hydrobromide solution is sprayed onto the microcrystalline cellulose at a rate of 80 g/minute until the microcrystalline cellulose contains, by weight of the layered particles, approximately 40% by weight of dextromethorphan hydrobromide.

### Part B: Preparation of Coated Layered Dextromethorphan

A coating solution is prepared containing Cellulose Acetate 398-10 (commercially available from Eastman Chemical, Kingsport, TN) and Eudragit E-100 (commercially available from Rohm America, , Piscataway, NJ) at a level of about 12% solids of Cellulose Acetate:Eudragit (80:20) in acetone (total solution weight equal to 10.7 kg).

A 3 kg portion of the particles prepared above in Part A is then charged into the rotor fluidized bed coating apparatus (Glatt Model GPCG 5/9). The particles are then fluidized with an airflow at 36°C, following which the coating solution is sprayed onto the particles at a rate of 40 g/minute until the drug particles contain approximately 20%, by weight, of the coating.

### Example 2: Preparation of Disintegrative Tablet Portion Containing Dextromethorphan

### Part A: Preparation of Disintegrative Tablet Portion Blend

All materials set forth in Table 1 below (except the coated dextromethorphan) are manually passed through a 30 mesh screen. 1.5 kg of the resulting blend and coated dextromethorphan of Example 1 are then placed into a 4 quart V-Blender and mixed for 5 minutes.

**Table 1: Components of Tablet Base Blend**

| Ingredients | Percent (w/w) | mg per tablet |
|---|---|---|
| Coated Dextromethorphan (32%)* | 13.7 | 90.45 |
| Crospovidone | 0.75 | 4.95 |
| Microcrystalline Cellulose (Avicel PH100) | 5 | 33 |
| Dextrose Monohydrate | 78.8 | 520.05 |
| Citric Acid USP | 0.5 | 3.3 |
| Peppermint Flavor | 0.5 | 3.3 |
| Magnesium Stearate | 0.75 | 4.95 |
| TOTAL | 100.0 | 660.0 |

| | | |
|---|---|---|
| * Equivalent to a 30 mg Dose of Dextromethorphan HBr. Coated dextromethorphan particles produced in accordance with Example 1. | | |

### Part B: Preparation of Concave Disintegrative Tablet Portion

400 g of the resulting blend of Part A above is removed from the blender and compressed on a rotary tablet press at 60 rpm using 7/16 inch extra deep convex tablet tooling in order to yield concave shaped tablets having a weight of 660 mg, a hardness range of about 3 to about 7 kp/cm², and a thickness of from about 0.3 to about 0.31 inches.

### Part C: Preparation of Flat Disintegrative Tablet Portion

400 g of the resulting blend of Part A above is removed from the blender and compressed on a rotary tablet press at 60 rpm using 7/16 inch flat faced tablet tooling in order to yield tablets having a weight of 660 mg and a hardness range of about 3 to about 7 kp/cm², and a thickness of about 0.25 to about 0.26 inches.

### Part D: Preparation of Convex Disintegrative Tablet Portion

400 g of the resulting blend of Part A above is removed from the blender and compressed on a rotary tablet press at 60 rpm using 7/16 inch extra deep concave tablet tooling in order to yield convex shaped tablets having a weight of 660 mg, a hardness range of from about 3 to about 7 kp/cm², and a thickness of from about 0.3 to about 0.31 inches.

### Example 3: Preparation of Dosage Form Containing Boiled Sugar Hard Candy Portion and Disintegrative Tablet Portion Core

A sugar hardy candy portion solution containing menthol is prepared using the materials outlined in below:

**Table 2: Components of Boiled Sugar Hard Candy Portion Blend**

| Ingredients | Percent (w/w) | mg per hard candy portion |
|---|---|---|
| Isomalt Sugar | 97.54 | 975.4 |
| Menthol USP | 1.2 | 12 |
| Red Coloring Dye #40 | 0.01 | 0.1 |
| Sucralose | 0.25 | 2.5 |
| Citric Acid USP | 0.5 | 5 |
| Cherry Flavor | 0.5 | 5 |
| Deionized Water | --- | --- |
| TOTAL | 100 | 1000 |

500 g of Isomalt Sugar and 75 g of Deionized Water are mixed in a stainless steel pot, and heated to 170°C until the water is evaporated. The mixture is then cooled to 140°C and the red coloring, citric acid, sucralose, menthol and cherry flavor are then added and mixed.

The compressed tablets from Example 2, Part B are then placed into a stainless steel mold, which covers the concave faces of the tablets. The mold also contains injection ports in that allow the above flowable hard candy portion blend to surround the circumference of the tablet at the bellyband of the tablet, but not the concave faces of the tablet. The hard candy portion blend, which is still heated at 140°C, is filled into a plastic syringe and manually injected to the mold, following which it is allowed to cool at room temperature for 15 minutes. The compressed core filled hard candy dosage forms are then removed from the mold. The resulting dosage forms provides for a taste-masked systemic delivery of dextromethorphan (a cough suppressant) and local delivery of a separate cough suppressant (menthol).

### Example 4: Preparation of Dual Layer Dosage Form Containing Boiled Sugar Hard Candy Portion Layer and Disintegrative Tablet Portion Layer

The hard candy portion blend from Example 3 is prepared as stated in the example. While in the flowable state, the hard candy portion blend is deposited using a 10-cc plastic syringe, into circular stainless steel molds with dual flat faces. The resulting hard candy portions are allowed to cool and harden at room temperature for approximately 15 minutes. The hard candy portion is then placed into a rubber mold. Approximately 30 milligrams of powdered polyethylene glycol (PEG) 3350 is evenly dispersed along one surface of the hard candy portion.

The flat faced compressed tablet from Example 2, Part C is then placed on top of the hard candy portion, and the resulting dosage form is placed into an oven set at 80°C for 30 minutes such that the PEG 3350 melted and created an adhesion between the compressed tablet and hard candy portion layers. The resulting dual layered dosage form is then allowed to cool at room temperature for 30 minutes and removed from the rubber mold.

### Example 5: Preparation of Dual Layer Dosage Form Containing Boiled Sugar Hard Candy Portion Layer and Disintegrative Tablet Portion Layer Having Concave/Convex Interface

The hard candy portion blend from Example 3 is prepared as stated in the example. While still in its flowable state, the hard candy portion is deposited into circular stainless steel molds with convex faces (which in turn formed a hard candy portion with a concave faces). The hard candy portions are allowed to cool and harden at room temperature for approximately 15 minutes. The hard candy portion is then placed into a rubber mold with the concave face facing in the upward position. Approximately 30 milligrams of polyethylene glycol (PEG) 3350 (which is previously melted at 80°C in a 50 mL stainless steel vessel) is placed on the surface of the hard candy portion face. The convex compressed tablet from Example 2, Part D is then placed on top of the hard candy portion such that the PEG could be displaced along the entire interface and create an adhesion between the compressed tablet and hard candy portion layers. The dual layered dosage form is then allowed to cool at room temperature for 10 minutes and removed from the rubber mold.

### Example 6 : Preparation of Compressed Sugar Hard Candy Portion Layer

All materials set forth in Table 3 below are manually passed through a 30 mesh screen. 1.5 kg of the resulting blend are placed into a 4 quart V-Blender and mixed for 5 minutes.

**Table 3: Components of Compressed Hard Candy Portion Blend**

| Ingredients | Percent (w/w) | mg/hard candy portion |
|---|---|---|
| Sorbitol | 5.00 | 50.0 |
| Compressible Sucrose * | 92.75 | 927.5 |
| Menthol | 1.00 | 10.0 |
| Peppermint Flavor | 0.50 | 5.0 |
| Magnesium Stearate | 0.75 | 7.5 |
| TOTAL | 100.0 | 1000.0 |

| | | |
|---|---|---|
| * Commercially available from Domino Specialty Ingredients, Baltimore, MD | | |

400 g of the resulting blend is then removed from the blender and compressed on a rotary tablet press at 60 rpm using 5/8" flat faced beveled edge (FFBE) tablet tooling in order to yield flat faced tablets having a weight of 1000 mg and a hardness range of not less than 15 kp/cm2, and a thickness of about 0.20 inches.

### Example 7: Preparation of Dual Layer Dosage Form Containing Compressed Sugar Hard Candy Portion Layer and Disintegrative Tablet Portion Layer

The compressed hard candy portion from Example 6 is placed into a rubber mold with the flat face facing upward. Approximately 30 milligrams of polyethylene glycol (PEG) 3350 which is melted at 80°C in a 50 mL stainless steel vessel is placed on the surface of the hard candy portion face. The flat faced tablet from Example 2, Part C is then placed on top of the hard candy portion, so that the PEG is displaced along the interface to create an adhesion between the compressed tablet and hard candy portion layers. The dual layered dosage form is then allowed to cool at room temperature for 10 minutes and removed from the rubber mold. This dosage form provides a taste-masked systemic delivery of dextromethorphan (a cough suppressant) and local delivery of a separate cough suppressant (menthol).

### Example 7 : Preparation of Bi-layer Dosage Form with Compressed Disintegrative Tablet Portion and a Compressed Sugar Hard Candy Portion

All materials set forth in Table 4 below are manually passed through a 30 mesh screen. 1.0 kg of the resulting blend are placed into a 4 quart V-Blender and mixed for 5 minutes.

**Table 4: Components of Compressed Hard Candy Layer Blend**

| Ingredient | mg/tab | g/batch |
|---|---|---|
| Compressible Isomalt* | 630 | 899.6 |
| Peppermint Flavor | 11 | 15.7 |
| Sucralose NF | 1 | 1.4 |
| Sodium Carbonate Anhydrous | 20 | 28.6 |
| Sodium Hydrogen Carbonate | 10 | 14.3 |
| Nicotine Resin Complex (20% nicotine) | 20 | 28.6 |
| D&C Red #7 Ca Lake | 0.3 | 0.4 |
| Mg Stearate NF | 8 | 11.4 |
| Lozenge Layer TOTAL | 700.3 | 1000 |

| | | |
|---|---|---|
| * Commercially available from Palatinit, Mannheim, Germany | | |

All materials set forth in Table 5 below are manually passed through a 30 mesh screen. 0.32 kg of the resulting blend are placed into a 1 quart V-Blender and mixed for 5 minutes.

**Table 5: Components of Compressed Disintegrative Layer Blend**

| Ingredient | mg/tab | g/batch |
|---|---|---|
| Sucralose NF | 1.5 | 2.1 |
| Cinnamon Flavor | 4.5 | 6.3 |
| Crospovidone NF | 15 | 21.1 |
| Dextrose Monohydrate | 200 | 281.9 |
| Mg Stearate NF | 6 | 8.5 |
| Disintegrative Tablet Layer TOTAL | 227 | 320 |

The resulting blends are then removed from the blenders and compressed on a bilayer rotary tablet press at 40 rpm using 1/2" diameter Troche tablet tooling in order to yield bilayer tablets having a weight of approximately 927.3 mg and a hardness range of not less than 15 kp/cm2, and a thickness of about 0.3 inches.

### Example 8: Preparation of Bi-layer Dosage Form with Compressed Disintegrative Tablet Portion and a Compressed Sugar Hard Candy Portion

All materials set forth in Table 6 below are manually passed through a 30 mesh screen. 1.0 kg of the resulting blend are placed into a 4 quart V-Blender and mixed for 5 minutes.

**Table 6: Components of Compressed Hard Candy Layer Blend**

| Ingredient | mg/tab | g/batch |
|---|---|---|
| Compressible Isomalt* | 839 | 559.3 |
| Peppermint Flavor | 12 | 8.0 |
| Vanilla Flavor | 1 | 0.7 |
| Sucralose NF | 1 | 0.7 |
| CaCO3 Granulation ** | 631.6 | 421.1 |
| FD&C Blue #1 HT Al Lake | 0.4 | 0.3 |
| Mg Stearate NF | 15 | 10.0 |
| Lozenge Layer TOTAL | 1500 | 1000 |

| | | |
|---|---|---|
| * Commercially available from Palatinit, Mannheim, Germany ** Commercially available from Particle Dynamics Inc., St. Louis, MO | | |

All materials set forth in Table 7 below are manually passed through a 30 mesh screen. 0.10 kg of the resulting blend are placed into a 1 quart V-Blender and mixed for 5 minutes.

**Table 7: Components of Compressed Disintegrative Layer Blend**

| Ingredient | mg/tabl | g/batch |
|---|---|---|
| Sucralose NF | 2 | 0.6 |
| Vanilla Flavor | 2.5 | 0.8 |
| Polyethylene Oxide NF | 5 | 1.6 |
| Crospovidone NF | 20 | 6.3 |
| Tastemasked Famotidine Granulation | 86.55 | 27.2 |
| Dextrose Monohydrate- Cerelose Grade 2033 | 200 | 62.8 |
| Mg Stearate NF (Grade 2257 Veg Source) | 2.5 | 0.8 |
| Disintegrative Tablet Layer TOTAL | 318.55 | 100 |

| | | |
|---|---|---|
| * Commercially available from McNeil CHC, Fort Washington, PA | | |

The resulting blends are then removed from the blenders and compressed on a bilayer rotary tablet press at 40 rpm using 5/8" diameter Troche tablet tooling in order to yield bilayer tablets having a weight of approximately 1818.55 mg and a hardness range of not less than 15 kp/cm2, and a thickness of about 0.35 inches.

### Example 9 : Preparation of Bi-layer Dosage Form with Compressed Disintegrative Tablet Portion and a Compressed Sugar Hard Candy Portion

All materials set forth in Table 8 below are manually passed through a 30 mesh screen. 1.0 kg of the resulting blend are placed into a 4 quart V-Blender and mixed for 5 minutes.

**Table 8: Components of Compressed Hard Candy Layer Blend**

| Ingredient | mg/tab | g/batch |
|---|---|---|
| Compressible Isomalt* | 1363.6 | 906.0 |
| Peppermint Flavor | 15 | 10.0 |
| Sucralose NF | 1 | 6.6 |
| Ibuprofen USP | 100 | 66.5 |
| Benzocaine | 5 | 3.3 |
| FD&C Blue # 1 HT Al Lake | 0.4 | 0.27 |
| Mg Stearate NF | 20 | 13.3 |
| Lozenge Layer TOTAL | 1505 | 1000 |

| | | |
|---|---|---|
| * Commercially available from Palatinit, Mannheim, Germany | | |

All materials set forth in Table 9 below are manually passed through a 30 mesh screen. 0.10 kg of the resulting blend are placed into a 1 quart V-Blender and mixed for 5 minutes.

**Table 9: Components of Compressed Disintegrative Layer Blend**

| Ingredient | mg/tab | g/batch |
|---|---|---|
| Sucralose NF | 2 | 0.84 |
| Vanilla Flavor | 2.5 | 1.04 |
| Polyethylene Oxide NF | 2.5 | 1.04 |
| Crospovidone NF | 20 | 8.35 |
| Benzocaine USP | 10 | 4.18 |
| Dextrose Monohydrate | 200 | 83.51 |
| Mg Stearate NF | 2.5 | 1.04 |
| Disintegrative Tablet Layer TOTAL | 239.5 | 100 |

The resulting blends are then removed from the blenders and compressed on a bilayer rotary tablet press at 40 rpm using 5/8" diameter Troche tablet tooling in order to yield bilayer tablets having a weight of approximately 1744.5 mg and a hardness range of not less than 15 kp/cm2, and a thickness of about 0.30 inches.

## Claims

1. A dosage form comprised of both a rapidly disintegrative tablet portion and a hard candy portion, wherein:
(i) the rapidly disintegrative tablet portion comprises at least one pharmaceutically active agent, and
(ii) the hard candy portion covers at least 20% of the surface of the disintegrative tablet portion, and
wherein the disintegration time of the hard candy portion is at least ten times longer than the disintegration time of the rapidly disintegrative tablet portion; and
wherein if the hard candy portion covers all of the surface area of the rapidly disintegrative tablet portion, then the hard candy portion further comprises a plurality of indentations that, upon contact with the fluids in the oral cavity, are adapted to dissolve and expose the surface area of the rapidly disintegrative tablet portion.

2. The dosage form of claim 1 wherein the rapidly disintegrative tablet portion has a hardness of less than 147 N/cm² (15 kp/cm²), and the hard candy portion has hardness of greater than 147 N/cm² (15 kp/cm²).

3. The dosage form of claim 1, wherein the pharmaceutically active agent comprised within the rapidly disintegrative tablet portion is selected from the group consisting of phenylephrine, dextromethorphan, pseudoephedrine, acetaminophen, ibuprofen, ketoprofen, loperamide, famotidine, calcium carbonate, simethicone, and menthol, and pharmaceutically acceptable salts thereof.

4. The dosage form of claim 1 wherein the pharmaceutically active agent is in the form of particles that are further coated with a taste-masking polymer and wherein the average particle diameter of the particles is from 50 µm (microns) to 1000 µm (microns).

5. The dosage form of claim 1, wherein the hard candy portion comprises a pharmaceutically active agent different from the pharmaceutically active agent comprised within the rapidly disintegrative tablet portion.

6. The dosage form of claim 5, wherein the pharmaceutically active agent comprised within the hard candy portion is selected from the group consisting of phenylephrine, dextromethorphan, pseudoephedrine, chlorpheniramine, methocarbomal, chlophedianol, ascorbic acid, menthol, pectin, dyclonine, and benzocaine, and pharmaceutically acceptable salts thereof.

7. A dosage form of claim 1 wherein the hard candy portion comprises at least 50%, by weight, of a sugar selected from the group consisting of isomalt, sucrose, dextrose, corn syrup, lactitol, and lycasin, and mixtures thereof.

8. The dosage form of claim 1, wherein the rapidly disintegrative tablet portion comprises at least 40 percent by weight of a compressible excipient selected from the group consisting of isomalt, dextrose monohydrate, maltodextrin, lactose monohydrate, dextrin, mannitol, lactitol, sorbitol, xylitol, erythritol, sucrose, and lactose, and mixtures thereof.

9. The dosage form of claim 8 wherein the compressible excipient is in the form of particles with an average particle diameter of 75 to 400 µm (microns).

10. A dosage form of claim 9, wherein the rapidly disintegrative tablet portion further comprises a water-swellable excipient selected from the group consisting of sodium starch glycolate, crospovidone, croscarmellose, microcrystalline cellulose, starches, hydroxypropyl cellulose, and alginic acid.

11. The dosage form of claim 9, wherein the weight ratio of the compressible excipient to the water-swellable excipient is from 10:1 1 to 100:1.

12. A dosage form of claim 7, wherein the rapidly disintegrative tablet portion further comprises a effervescent couple comprising one member selected from the group consisting of sodium bicarbonate, potassium bicarbonate, calcium carbonate, magnesium carbonate, and sodium carbonate and one member selected from the group consisting of citric acid, malic acid, fumaric acid, tartaric acid, and alginic acid.

13. The dosage form of claim 1, wherein the weight ratio between the rapidly disintegrative tablet portion and a hard candy portion is from 10:90 to 60:40.

14. A dosage form of claim 1, wherein the hard candy portion covers at least 50 percent of the surface area of the rapidly disintegrative tablet portion.

15. A dosage portion of claim 14, wherein the hard candy portion comprises a plurality of openings exposing the surface area of the rapidly disintegrative tablet portion.

16. The dosage form of claim 14, wherein the hard candy portion covers all of the surface area of the rapidly disintegrative tablet portion and wherein the hard candy portion further comprises a plurality of indentations that, upon contact with the fluids in the oral cavity, are adapted to dissolve and expose the surface area of the rapidly disintegrative tablet portion.

17. The dosage form of claim 1, wherein the rapidly disintegrative tablet portion has multiple layers comprising at least one different ingredient.

18. The dosage form of claim 17, wherein said rapidly disintegrative tablet portion comprises two layers, wherein the first layer comprises the pharmaceutically active agent and a second layer comprises a second pharmaceutically active agent that may be different from the pharmaceutically active agent comprised within the first layer.

19. The dosage form of claim 17, wherein said rapidly disintegrative tablet portion comprises two layers, wherein both the first layer and the second comprises the pharmaceutically active agent and further wherein pharmaceutically active agent within the second layer is coated with a sustained release coating.

20. The dosage form of claim 17, wherein both the first layer and the second layer are exposed on the surface of the dosage form.

21. The dosage form of claim 1, wherein the dosage form has a multiple layer structure, wherein the rapidly disintegrative tablet portion is a first layer and the hard candy portion is a second layer.

22. The dosage form of claim 21, wherein the face of one layer has a convex shape and the face of the other layer has a concave shape.

23. The dosage form of claim 21, further comprising a third layer positioned between a face of the first layer and a face of the second layer, wherein the third layer comprises an edible adhesive-like material.

24. The dosage form of claim 23, wherein the edible adhesive-like material comprises an ingredient selected from the group consisting of polyethylene glycol, polyethylene oxide, polycaprolactone, carnuba wax, microcrystalline wax, oppanol, shellac wax and beeswax.

25. The dosage form of claim 1 for use in therapy.

26. The dosage form of claim 25, wherein the rapidly disintegrative tablet portion comprises at least one pharmaceutically active agent selected from the group of phenylephrine, dextromethorphan, chlorpheniramine, chlophedianol, and pseudoephedrine and the hard candy portion comprises at least one pharmaceutically active agent selected from the group of menthol, nicotine, dyclonine, pectin, and benzocaine.

27. The dosage form of claim 1 for use in a method of treating an ailment selected from pain, fever, inflammation, upper respiratory disorders, infections, depression, diabetes, obesity, cardiovascular disorders, gastrointestinal disorders, sleep disorders, osteoporosis, and nicotine dependence, the method comprising orally administering the dosage form of claim 1 wherein the dosage form comprises an amount of the pharmaceutically active agent effective to treat the ailment.

## Patentansprüche

1. Dosierungsform, zusammengesetzt aus einem schnell zerfallenden Tablettenteil und einem harten Konfektteil, wobei:
(i) der schnell zerfallende Tablettenteil wenigstens einen pharmazeutischen Wirkstoff umfasst, und
(ii) der harte Konfektteil wenigstens 20 % der Oberfläche des zerfallenden Tablettenteils bedeckt, und
wobei die Zerfallszeit des harten Konfektteils wenigstens zehnmal länger als die Zerfallszeit des schnell zerfallenden Tablettenteils ist; und
wobei, wenn der harte Konfektteil die gesamte Oberfläche des schnell zerfallenden Tablettenteils bedeckt, der harte Konfektteil ferner eine Vielzahl von Vertiefungen umfasst, die dafür ausgelegt sind, sich bei Kontakt mit den Fluiden in der Mundhöhle aufzulösen und die Oberfläche des schnell zerfallenden Tablettenteils zu exponieren.

2. Dosierungsform gemäß Anspruch 1, wobei der schnell zerfallende Tablettenteil eine Härte von weniger als 147 N/cm² (15 kp/cm²) aufweist und der harte Konfektteil eine Härte von mehr als 147 N/cm² (15 kp/cm²) aufweist.

3. Dosierungsform gemäß Anspruch 1, wobei der pharmazeutische Wirkstoff, der in dem schnell zerfallenden Tablettenteil enthalten ist, ausgewählt ist aus der Gruppe bestehend aus Phenylephrin, Dextromethorphan, Pseudoephedrin, Acetaminophen, Ibuprofen, Ketoprofen, Loperamid, Famotidin, Calciumcarbonat, Simethicon und Menthol und pharmazeutisch verträglichen Salzen davon.

4. Dosierungsform gemäß Anspruch 1, wobei der pharmazeutische Wirkstoff in der Form von Partikeln vorliegt, die ferner mit einem geschmacksmaskierenden Polymer überzogen sind und wobei der mittlere Partikeldurchmesser der Partikel 50 µm (Mikrometer) bis 1000 µm (Mikrometer) beträgt.

5. Dosierungsform gemäß Anspruch 1, wobei der harte Konfektteil einen pharmazeutischen Wirkstoff umfasst, der von dem in dem schnell zerfallenden Tablettenteil enthaltenen pharmazeutischen Wirkstoff verschieden ist.

6. Dosierungsform gemäß Anspruch 5, wobei der pharmazeutische Wirkstoff, der in dem harten Konfektteil enthalten ist, ausgewählt ist aus der Gruppe bestehend aus Phenylephrin, Dextromethorphan, Pseudoephedrin, Chlorpheniramin, Methocarbomal, Chlophedianol, Ascorbinsäure, Menthol, Pectin, Dyclonin und Benzocain und pharmazeutisch verträglichen Salzen davon.

7. Dosierungsform gemäß Anspruch 1, wobei der harte Konfektteil wenigstens 50 Ges.-% an einem Zucker umfasst, ausgewählt aus der Gruppe bestehend aus Isomalt, Sucrose, Dextrose, Maissirup, Lactitol und Lycasin und Gemischen davon.

8. Dosierungsform gemäß Anspruch 1, wobei der schnell zerfallende Tablettenteil wenigstens 40 Gewichtsprozent an einem komprimierbaren Hilfsstoff umfasst, ausgewählt aus der Gruppe bestehend aus Isomalt, Dextrosemonohydrat, Maltodextrin, Lactosemonohydrat, Dextrin, Mannitol, Lactitol, Sorbitol, Xylitol, Erythritol, Sucrose und Lactose und Gemischen davon.

9. Dosierungsform gemäß Anspruch 8, wobei der komprimierbare Hilfsstoff in der Form von Partikeln mit einem mittleren Partikeldurchmesser von 75 bis 400 µm (Mikrometer) vorliegt.

10. Dosierungsform gemäß Anspruch 9, wobei der schnell zerfallende Tablettenteil ferner einen wasserquellbaren Hilfsstoff umfasst, ausgewählt aus der Gruppe bestehend aus Natriumstärkeglykolat, Crospovidon, Croscarmellose, mikrokristalliner Cellulose, Stärken, Hydroxypropylcellulose und Alginsäure.

11. Dosierungsform gemäß Anspruch 9, wobei das Gewichtsverhältnis des komprimierbaren Hilfsstoffs zu dem wasserquellbaren Hilfsstoff 10:1 bis 100:1 beträgt.

12. Dosierungsform gemäß Anspruch 7, wobei der schnell zerfallende Tablettenteil ferner ein Brausepaar umfasst, umfassend einen Bestandteil, ausgewählt aus der Gruppe bestehend aus Natriumbicarbonat, Kaliumbicarbonat, Calciumcarbonat, Magnesiumcarbonat und Natriumcarbonat, und einen Bestandteil, ausgewählt aus der Gruppe bestehend aus Citronensäure, Äpfelsäure, Fumarsäure, Weinsäure und Alginsäure.

13. Dosierungsform gemäß Anspruch 1, wobei das Gewichtsverhältnis zwischen dem schnell zerfallenden Tablettenteil und dem harten Konfektteil 10:90 bis 60:40 beträgt.

14. Dosierungsform gemäß Anspruch 1, wobei der harte Konfektteil wenigstens 50 Prozent der Oberfläche des schnell zerfallenden Tablettenteils bedeckt.

15. Dosierungsform gemäß Anspruch 14, wobei der harte Konfektteil eine Vielzahl von Öffnungen umfasst, die die Oberfläche des schnell zerfallenden Tablettenteils exponieren.

16. Dosierungsform gemäß Anspruch 14, wobei der harte Konfektteil die gesamte Oberfläche des schnell zerfallenden Tablettenteils bedeckt und wobei der harte Konfektteil ferner eine Vielzahl von Vertiefungen umfasst, die dafür ausgelegt sind, sich bei Kontakt mit den Fluiden in der Mundhöhle aufzulösen und die Oberfläche des schnell zerfallenden Tablettenteils zu exponieren

17. Dosierungsform gemäß Anspruch 1, wobei der schnell zerfallende Tablettenteil mehrere Schichten aufweist, die wenigstens einen unterschiedlichen Inhaltsstoff umfassen.

18. Dosierungsform gemäß Anspruch 17, wobei der schnell zerfallende Tablettenteil zwei Schichten umfasst, wobei die erste Schicht den pharmazeutischen Wirkstoff umfasst und eine zweite Schicht einen zweiten pharmazeutischen Wirkstoff umfasst, der von dem in der ersten Schicht enthaltenen pharmazeutischen Wirkstoff verschieden sein kann.

19. Dosierungsform gemäß Anspruch 17, wobei der schnell zerfallende Tablettenteil zwei Schichten umfasst, wobei sowohl die erste Schicht als auch die zweite Schicht den pharmazeutischen Wirkstoff umfassen und wobei ferner der in der zweiten Schicht enthaltene pharmazeutische Wirkstoff mit einem Überzug zur anhaltenden Freisetzung überzogen ist.

20. Dosierungsform gemäß Anspruch 17, wobei sowohl die erste Schicht als auch die zweite Schicht an der Oberfläche der Dosierungsform exponiert ist.

21. Dosierungsform gemäß Anspruch 1, wobei die Dosierungsform eine Mehrschichtstruktur aufweist, wobei der schnell zerfallende Tablettenteil eine erste Schicht ist und der harte Konfektteil eine zweite Schicht ist.

22. Dosierungsform gemäß Anspruch 21, wobei die Oberfläche einer Schicht eine konvexe Form und die Oberfläche der anderen Schicht eine konkave Form aufweist.

23. Dosierungsform gemäß Anspruch 21, ferner umfassend eine dritte Schicht, die zwischen einer Oberfläche der ersten Schicht und einer Oberfläche der zweiten Schicht angeordnet ist, wobei die dritte Schicht ein essbares, klebstoffartiges Material umfasst.

24. Dosierungsform gemäß Anspruch 23, wobei das essbare, klebstoffartige Material einen Inhaltsstoff umfasst, ausgewählt aus der Gruppe bestehend aus Polyethylenglykol, Polyethylenoxid, Polycaprolacton, Carnaubawachs, mikrokristallinem Wachs, Oppanol, Schellackwachs und Bienenwachs.

25. Dosierungsform gemäß Anspruch 1 für die Verwendung bei der Therapie.

26. Dosierungsform gemäß Anspruch 25, wobei der schnell zerfallende Tablettenteil wenigstens einen pharmazeutischen Wirkstoff umfasst, ausgewählt aus der Gruppe von Phenylephrin, Dextromethorphan, Chlorpheniramin, Chlorphedianol und Pseudoephedrin, und der harte Konfektteil wenigstens einen pharmazeutischen Wirkstoff umfasst, ausgewählt aus der Gruppe von Menthol, Nicotin, Dyclonin, Pectin und Benzocain.

27. Dosierungsform gemäß Anspruch 1 für die Verwendung bei einem Verfahren zum Behandeln eines Leidens, ausgewählt aus Schmerz, Fieber, Entzündung, Störungen der oberen Atemwege, Infektionen, Depression, Diabetes, Adipositas, kardiovaskulären Störungen, Magen-Darm-Störungen, Schlafstörungen, Osteoporose und Nikotinabhängigkeit, wobei das Verfahren das orale Verabreichen der Dosierungsform gemäß Anspruch 1 umfasst, wobei die Dosierungsform eine Menge des pharmazeutischen Wirkstoffs umfasst, die zum Behandeln des Leidens wirksam ist.

## Revendications

1. Forme pharmaceutique comprenant à la fois une partie comprimé à délitement rapide et une partie bonbon dur, **caractérisée en ce que**
(i) la partie comprimé à délitement rapide comprend au moins un agent pharmaceutiquement actif, et
(ii) la partie bonbon dur couvre au moins 20 % de la surface de la partie comprimé délitant, et
**en ce que** le temps de délitement de la partie bonbon dur est au moins dix fois plus lent que le temps de délitement de la partie comprimé à délitement rapide ; et
**en ce que** si la partie bonbon dur couvre toute la surface de la partie comprimé à délitement rapide, alors la partie bonbon dur comprend en outre une pluralité d'empreintes qui, au contact avec les liquides dans la cavité buccale, sont adaptées pour se dissoudre et exposer la surface de la partie comprimé à délitement rapide.

2. Forme pharmaceutique selon la revendication 1, **caractérisée en ce que** la partie comprimé à délitement rapide a une dureté inférieure à 147 N/cm² (15 kp/cm²), et la partie bonbon dur a une dureté supérieure à 147 N/cm² (15 kp/cm²).

3. Forme pharmaceutique selon la revendication 1, **caractérisée en ce que** l'agent pharmaceutiquement actif contenu dans la partie comprimé à délitement rapide est choisi dans le groupe constitué par la phényléphrine, le dextrométhorphane, la pseudoéphédrine, l'acétaminophène, l'ibuprofène, le kétoprofène, le lopéramide, la famotidine, le carbonate de calcium, la siméthicone, et le menthol, et les sels pharmaceutiquement acceptables de ceux-ci.

4. Forme pharmaceutique selon la revendication 1, **caractérisée en ce que** l'agent pharmaceutiquement actif est sous forme de particules qui sont en outre revêtues d'un polymère masqueur de goût et **en ce que** le diamètre moyen des particules est de 50 µm (microns) à 1000 µm (microns).

5. Forme pharmaceutique selon la revendication 1, **caractérisée en ce que** la partie bonbon dur comprend un agent pharmaceutiquement actif différent de l'agent pharmaceutiquement actif contenu dans la partie comprimé à délitement rapide.

6. Forme pharmaceutique selon la revendication 5, **caractérisée en ce que** l'agent pharmaceutiquement actif contenu dans la partie bonbon dur est choisi dans le groupe constitué par la phényléphrine, le dextrométhorphane, la pseudoéphédrine, la chlorophéniramine, le méthocarbomal, le chlophédianol, l'acide ascorbique, le menthol, la pectine, la dyclonine, et la benzocaïne, et les sels pharmaceutiquement acceptables de ceux-ci.

7. Forme pharmaceutique selon la revendication 1, **caractérisée en ce que** la partie bonbon dur comprend au moins 50 % en poids d'un sucre choisi dans le groupe constitué par l'isomalt, le saccharose, le dextrose, le sirop de maïs, le lactitol, et la lycasine, et des mélanges de ceux-ci.

8. Forme pharmaceutique selon la revendication 1, **caractérisée en ce que** la partie comprimé à délitement rapide comprend au moins 40 % en poids d'un excipient compressible choisi dans le groupe constitué par l'isomalt, le dextrose monohydraté, la maltodextrine, le lactose monohydraté, la dextrine, le mannitol, le lactitol, le sorbitol, le xylitol, l'érythritol, le saccharose, et le lactose, et les mélanges de ceux-ci.

9. Forme pharmaceutique selon la revendication 8, **caractérisée en ce que** l'excipient compressible est sous forme de particules ayant un diamètre moyen des particules de 75 à 400 µm (microns).

10. Forme pharmaceutique selon la revendication 9, **caractérisée en ce que** la partie comprimé à délitement rapide comprend en outre un excipient gonflable dans l'eau, choisi dans le groupe constitué par le glycolate d'amidon sodique, la crospovidone, la croscarmellose, la cellulose microcristalline, les amidons, l'hydroxypropylcellulose, et l'acide alginique.

11. Forme pharmaceutique selon la revendication 9, **caractérisée en ce que** le rapport en poids entre l'excipient compressible et l'excipient gonflable dans l'eau est de 10:1 à 100:1.

12. Forme pharmaceutique selon la revendication 7, **caractérisée en ce que** la partie comprimé à délitement rapide comprend en outre une paire effervescente comprenant un membre choisi dans le groupe constitué par le bicarbonate de sodium, le bicarbonate de potassium, le carbonate de calcium, le carbonate de magnésium, et le carbonate de sodium et un membre choisi dans le groupe constitué par l'acide citrique, l'acide malique, l'acide fumarique, l'acide tartrique et l'acide alginique.

13. Forme pharmaceutique selon la revendication 1, **caractérisée en ce que** le rapport en poids entre la partie comprimé à délitement rapide et une partie bonbon dur est de 10:90 à 60:40.

14. Forme pharmaceutique selon la revendication 1, **caractérisée en ce que** la partie bonbon dur couvre au moins 50 % de la surface de la partie comprimé à délitement rapide.

15. Forme pharmaceutique selon la revendication 14, **caractérisée en ce que** la partie bonbon dur comprend une pluralité d'ouvertures exposant la surface de la partie comprimé à délitement rapide.

16. Forme pharmaceutique selon la revendication 14, **caractérisée en ce que** la partie bonbon dur couvre toute la surface de la partie comprimé à délitement rapide et **en ce que** la partie bonbon dur comprend en outre une pluralité d'empreintes qui, au contact avec les liquides dans la cavité buccale, sont adaptées pour se dissoudre et exposer la surface de la partie comprimé à délitement rapide.

17. Forme pharmaceutique selon la revendication 1, **caractérisée en ce que** la partie comprimé à délitement rapide a plusieurs couches comprenant au moins un ingrédient différent.

18. Forme pharmaceutique selon la revendication 17, **caractérisée en ce que** ladite partie comprimé à délitement rapide comprend deux couches, la première couche comprenant l'agent pharmaceutiquement actif et une deuxième couche comprenant un deuxième agent pharmaceutiquement actif qui peut être différent de l'agent pharmaceutiquement actif contenu dans la première couche.

19. Forme pharmaceutique selon la revendication 17, **caractérisée en ce que** ladite partie comprimé à délitement rapide comprend deux couches, à la fois la première couche et la deuxième couche comprenant l'agent pharmaceutiquement actif et en outre l'agent pharmaceutiquement actif dans la deuxième couche étant revêtu par un revêtement à libération prolongée.

20. Forme pharmaceutique selon la revendication 17, **caractérisée en ce que** à la fois la première couche et la deuxième couche sont exposées sur la surface de la forme pharmaceutique.

21. Forme pharmaceutique selon la revendication 1, **caractérisée en ce que** la forme pharmaceutique a une structure à plusieurs couches, la partie comprimé à délitement rapide étant une première couche et la partie bonbon dur étant une deuxième couche.

22. Forme pharmaceutique selon la revendication 21, **caractérisée en ce que** la face d'une couche a une forme convexe et la face de l'autre couche a une forme concave.

23. Forme pharmaceutique selon la revendication 21, comprenant en outre une troisième couche positionnée entre une face de la première couche et une face de la deuxième couche, la troisième couche comprenant une matière de type adhésif comestible.

24. Forme pharmaceutique selon la revendication 23, **caractérisée en ce que** la matière de type adhésif comestible comprend un ingrédient choisi dans le groupe constitué par le polyéthylèneglycol, l'oxyde de polyéthylène, la polycaprolactone, la cire de carnuba, la cire microcristalline, l'oppanol, la cire shellac et la cire d'abeille.

25. Forme pharmaceutique selon la revendication 1, destinée à être utilisée en thérapie.

26. Forme pharmaceutique selon la revendication 25, **caractérisée en ce que** la partie comprimé à délitement rapide comprend au moins un agent pharmaceutiquement actif choisi dans le groupe comprenant la phényléphrine, le dextrométhorphane, la chlorophéniramine, le chlophédianol, et la pseudoéphédrine, et la partie bonbon dur comprend au moins un agent pharmaceutiquement actif choisi dans le groupe comprenant le menthol, la nicotine, la dyclonine, la pectine, et la benzocaïne.

27. Forme pharmaceutique selon la revendication 1, destinée à être utilisée dans une méthode de traitement d'une affection choisie parmi les douleurs, la fièvre, les inflammations, les troubles des voies respiratoires supérieures, les infections, la dépression, le diabète, l'obésité, les troubles cardiovasculaires, les troubles gastro-intestinaux, les troubles du sommeil, l'ostéoporose, et la dépendance à la nicotine, la méthode comprenant l'administration par voie orale de la forme pharmaceutique selon la revendication 1, la forme pharmaceutique comprenant une quantité de l'agent pharmaceutiquement actif efficace pour traiter l'affection.
